# EUROPEAN PATENT APPLICATION

(11) **EP 3 195 956 A1**
(43) Date of publication of application: **26.07.2017**
(21) Application number: 16190665.6
(22) Date of filing: 13.04.2013
(51) Int. Cl.: B22F 3/105, A61F 2/38, A61F 2/36, A61F 2/30, B22F 5/00

(54) **DEVICES AND METHODS FOR ADDITIVE MANUFACTURING OF IMPLANT COMPONENTS**

(30) Priority: 13.04.2012 US 201261623776 P
(62) Divisional of application: 13775348.9
(71) Applicant: ConforMIS, Inc., Bedford, MA 01730 (US)
(72) Inventor: MILLER, Bob, Secaucus, NJ 07094 (US); HESKETH, David, Methuen, MA 01844 (US)
(74) Representative: Grund, Martin

(57) **Abstract**

Improved devices and methods for additive manufacturing of implant components are disclosed, including improvements relating to utilizing support structures, aligning implant designs within the manufacturing apparatus, and making patient-adapted implants. In particular, support structures having areas of reduced cross-section (170, 220) are disclosed.

## Description

### RELATED APPLICATIONS

This application claims the benefit of U.S. Provisional Application Serial No. 61/623,776, entitled "Devices and Methods for Additive Manufacturing of Implant Components" and filed April 13, 2012, the disclosure of which is incorporated herein by reference in its entirety.

### TECHNICAL FIELD

Embodiments described herein relate to devices and methods for manufacturing implants, implant components and/or related tools using additive metals technologies, including SLM (selective laser melting) technologies. More specifically, various embodiments described herein include methods for improving the SLM manufacture of femoral implant components forming a portion of a patient-adapted knee joint implant.

### BACKGROUND

Historically, diseased, injured or defective joints, such as, for example, joints exhibiting osteoarthritis, were repaired using standard off-the-shelf implants and other surgical devices. Surgical implant systems that employed a one-size-fits-all approach to implant design (and even those that utilized a "few-sizes-fit-all" approach, including modularly assembled systems) did not typically require highly accurate information about the patient's anatomy. Instead, such systems utilized gross anatomical measurements such as the maximum bone dimensions at the implant site, as well as the patient weight and age, to determine a "suitable" implant. The surgical procedure then concentrated on altering the underlying bony anatomical support structures (e.g., by cutting, drilling and/or otherwise modifying the bone structures) to accommodate the existing contact surfaces of the pre-manufactured implant. With these systems, varying quantities of implants and/or implant components would be manufactured and stockpiled. Once a potential patient was identified, an appropriate implant and/or component would be selected, transported to the surgical location and utilized in the patient's surgical procedure.

More recently, the joint replacement field has come to embrace the concept of "patient-adapted" (i.e., "patient-specific" and/or "patient-engineered") implant systems. With such systems, the surgical implants, associated surgical tools and procedures are designed or otherwise modified to account for and accommodate the individual anatomy of the patient undergoing the surgical procedure. Such systems typically utilize non-invasive imaging data, taken of the individual preoperatively, to guide the design and/or selection of the implant, surgical tools, and the planning of the surgical procedure itself. Various objectives of these newer systems include (1) reducing the amount of bony anatomy removed to accommodate the implant, (2) designing/selecting an implant that replicates and/or improves the function of the natural joint, (3) increasing the durability and functional lifetime of the implant, (4) simplifying the surgical procedure for the surgeon, (5) reducing patient recovery time and/or discomfort, and (6) improving patient outcomes.

Because "patient-specific" and "patient-engineered" implant systems are created using anatomical information from a particular patient, such systems are generally created after the patient has been designated a "surgical candidate" and undergone non-invasive imaging. But, because such systems are not generally pre-manufactured and stockpiled in multiple sizes (as are traditional systems), there can be a considerable delay between patient diagnosis and the actual surgery, much of which is due to the amount of time necessary to design and manufacture the "patient-specific" and/or "patient-engineered" implant components using patent image data.

A significant portion of any delay between patient diagnosis/imaging and actual surgery can often be attributed to the time needed to manufacture each "patient-specific" and/or "patient-engineered" implant system to a particular patient's anatomy. Usually, such implants are manufactured individually or in small batches, using a 3rd party vendor, which can greatly increase the cost of creating such implant components as compared to the large batch manufacturing used with traditional non-custom implants.

In addition, because "patient-specific" and/or "patient-engineered" implant systems are manufactured in limited quantities, a fracture, failure or sufficient discrepancy identified at any point in the manufacturing process can have significant consequences, including the non-availability of implant components when needed and/or a requirement to remanufacture implant components and/or ordering implants on an expedited (and much more expensive) basis to meet deadlines.

Accordingly, there is a need in the art for advanced methods, techniques, devices and systems to ensure the availability of "patient-specific" and/or "patient-engineered" implant components for a scheduled surgery in a cost effective and efficient manner.

### SUMMARY

The embodiments described herein include advancements and improvements in or related to the use of additive manufacturing techniques, including Selective Laser Melting (SLM) manufacturing techniques, in the design, selection, development, manufacturing and/or finishing of patient-specific and/or patient-engineered implant components. Various embodiments described herein facilitate the production of "patient-specific" or "patient-engineered" implants in a more cost effective and/or efficient manner.

Various embodiments described herein include methods for improving the strength, quality, performance and/or durability of implant components manufactured using SLM or similar material-additive manufacturing techniques.

Various embodiments described herein include methods of improving and/or simplifying the post-manufacture processing and/or "finishing' of an implant component manufactured using SLM or similar material-additive manufacturing techniques.

Various embodiments described herein include methods of assessing and/or optimizing SLM manufacturing methods and/or modifying implant design features to accommodate different limitations associated with SLM manufacturing techniques and processes.

It is to be understood that the features of the various embodiments described herein are not mutually exclusive and may exist in various combinations and permutations.

### BRIEF DESCRIPTION OF THE DRAWINGS

Objects, aspects, features, and advantages of various embodiments will become more apparent and may be better understood by referring to the following description, taken in conjunction with the accompanying drawings, in which:
FIG. 1 depicts a schematic view of SLM equipment;
FIG. 2A is a perspective view of a femoral implant;
FIG. 2B is a perspective view of a femoral implant;
FIG. 3 is a perspective view of a femoral implant and support structures;
FIG. 4 is a close-up perspective view of a femoral implant manufactured with support structures;
FIG. 5 depicts a side view of support structures extending between implant posts;
FIG. 6A depicts a side view of support structures extending between implant posts;
FIG. 6B is a perspective view of a femoral implant and support structures;
FIG. 7 depicts a side view of support structures extending between implant posts;
FIG. 8 depicts a side view of support structures extending between implant posts;
FIG. 9 depicts various peg/post designs;
FIG. 10 depicts a cross-sectional view of an exemplary embodiment of an implant design and manufacturing orientation;
FIG. 11 depicts a cross-sectional view of an exemplary embodiment of an implant design and manufacturing orientation;
FIG. 12 depicts a cross-sectional view of an exemplary embodiment of an implant design and manufacturing orientation; and
FIG. 13 depicts a cross-sectional view of an exemplary embodiment of an implant design and manufacturing orientation.

Additional figure descriptions are included in the text below. Unless otherwise denoted in the description for each figure, "M" and "L" in certain figures indicate medial and lateral sides of the view, respectively; "A" and "P" in certain figures indicate anterior and posterior sides of the view, respectively; and "S" and "I" in certain figures indicate superior and inferior sides of the view, respectively.

### DETAILED DESCRIPTION

A number of significant challenges face the widespread adoption of patient-specific implants and associated surgical procedures, many of which relate to the amount of time required to manufacture the implant, as well as the significant costs associated with creating a unique implant for each individual surgical patient. Unlike standard and/or modular implants, which can be manufactured in bulk and stored for use as needed, patient-specific implants are generally created after a patient has been identified as a surgical candidate, and the implant is designed and/or selected using imaging data taken of the intended patient's anatomy. The process of designing, manufacturing and finishing the implant can involve a number of steps, typically involving multiple vendors, and this process must result in an acceptable implant before the surgery can occur. In some cases, traditional methods of creating of a patient-specific implant from patient imaging data can require more than 4 to 7 weeks, which is a significant delay for both the surgeon and the patient.

An additional challenge facing the acceptance of patient-specific implants relates to the significant costs associated with creating a unique implant for each individual patient. The unique nature of each patient-specific implant does not lend their creation to bulk manufacturing methods including high-volume casting techniques. Rather, individual implant components are generally designed and investment cast on an individual basis, or can be designed and machined from bulk raw materials, which can be a time-consuming and expensive process.

An additional concern relating to the use of patient-specific implants relates to the availability of processing and manufacturing equipment, as well as the assurance that the implant components will be processed and available for the surgical procedure. Because each patient-specific implant is unique, and because a significant amount of time and effort is required to create each implant, it is typical practice to manufacture multiple copies (e.g., a primary and a backup implant) of an implant for a single patient, to ensure that at least one implant survives the manufacturing, finishing and testing processes prior to surgical use. However, because such backup implants are only needed where the primary implant has failed, the constant creation of backup implants leads to unused inventory and unnecessary costs where the primary implant does not get damaged. In addition, creating a backup patient-specific implant often leads to significant wastage where the primary implant is deemed acceptable (which occurs in the vast majority of cases), as the backup implant is generally useless for any other patient and/or procedure and is typically scrapped. Moreover, there are occasions where the primary and back-up implant castings are both damaged, fractured and/or undergo processing missteps that render both implants useless, and there may not be an opportunity to remanufacture another suitable implant within a desired timeframe (or at a desired cost without significant expedited processing fees) for a variety of reasons, which can include a lack of personnel, equipment and/or unavailability of raw materials to create a replacement.

Various technologies appropriate for manufacturing implants and tools are known in the art, for example, as described in Wohlers Report 2009, State of the Industry Annual Worldwide Progress Report on Additive Manufacturing, Wohlers Associates, 2009 (ISBN 0-9754429-5-3), available from the web www.wohlersassociates.com; Pham and Dimov, Rapid manufacturing, Springer-Verlag, 2001 (ISBN 1-85233-360-X); Grenda, Printing the Future, The 3D Printing and Rapid Prototyping Source Book, Castle Island Co., 2009; Virtual Prototyping & Bio Manufacturing in Medical Applications, Bidanda and Bartolo (Eds.), Springer, December 17, 2007 (ISBN: 10: 0387334297; 13: 978-0387334295); Bio-Materials and Prototyping Applications in Medicine, Bártolo and Bidanda (Eds.), Springer, December 10, 2007 (ISBN: 10: 0387476822; 13: 978-0387476827); Liou, Rapid Prototyping and Engineering Applications: A Toolbox for Prototype Development, CRC, September 26, 2007 (ISBN: 10: 0849334098; 13: 978-0849334092); Advanced Manufacturing Technology for Medical Applications: Reverse Engineering, Software Conversion and Rapid Prototyping, Gibson (Ed.), Wiley, Jan. 2006 (ISBN: 10: 0470016884; 13: 978-0470016886); and Branner et al., "Coupled Field Simulation in Additive Layer Manufacturing," 3rd International Conference PMI, 2008 (10 pages).

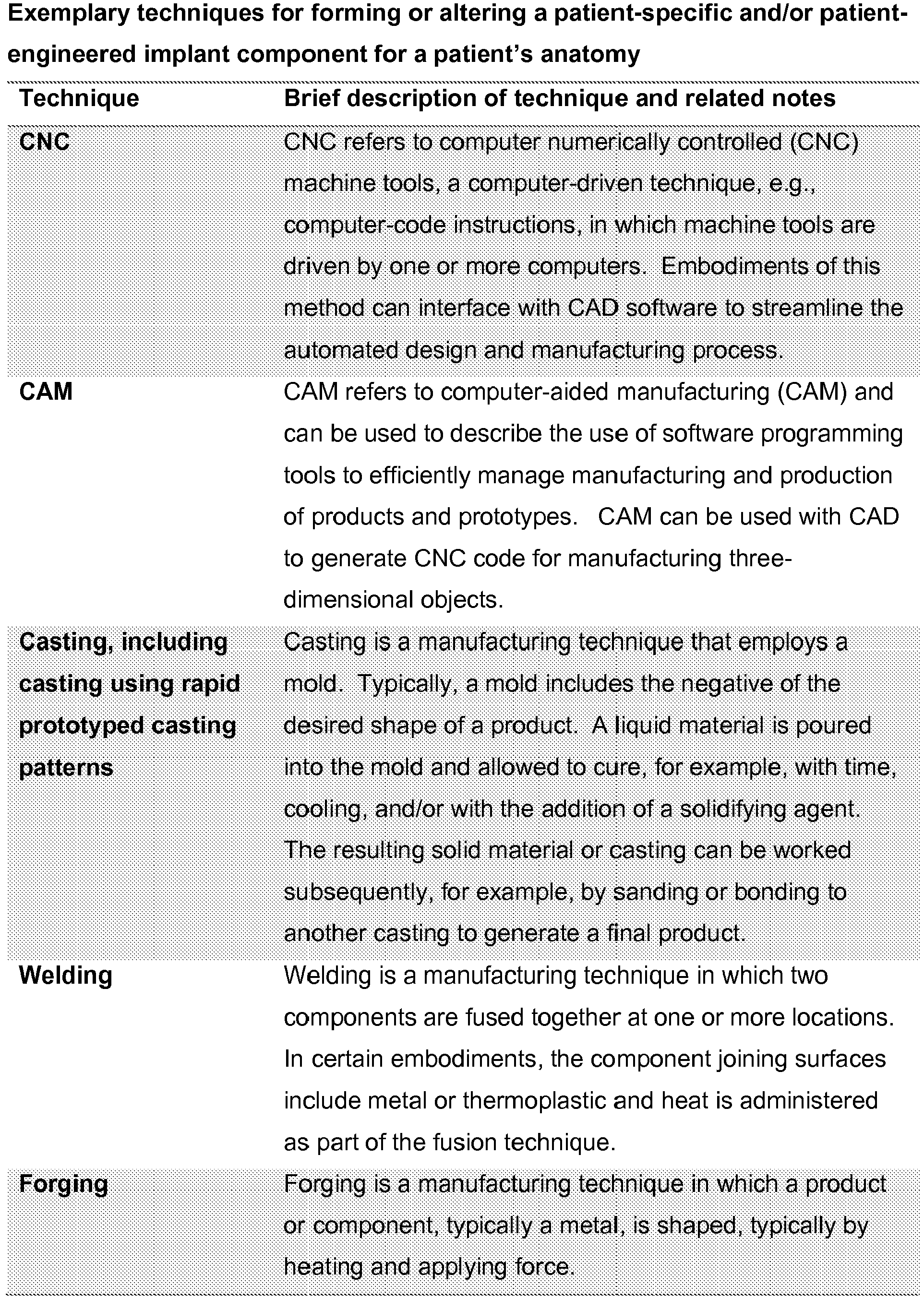

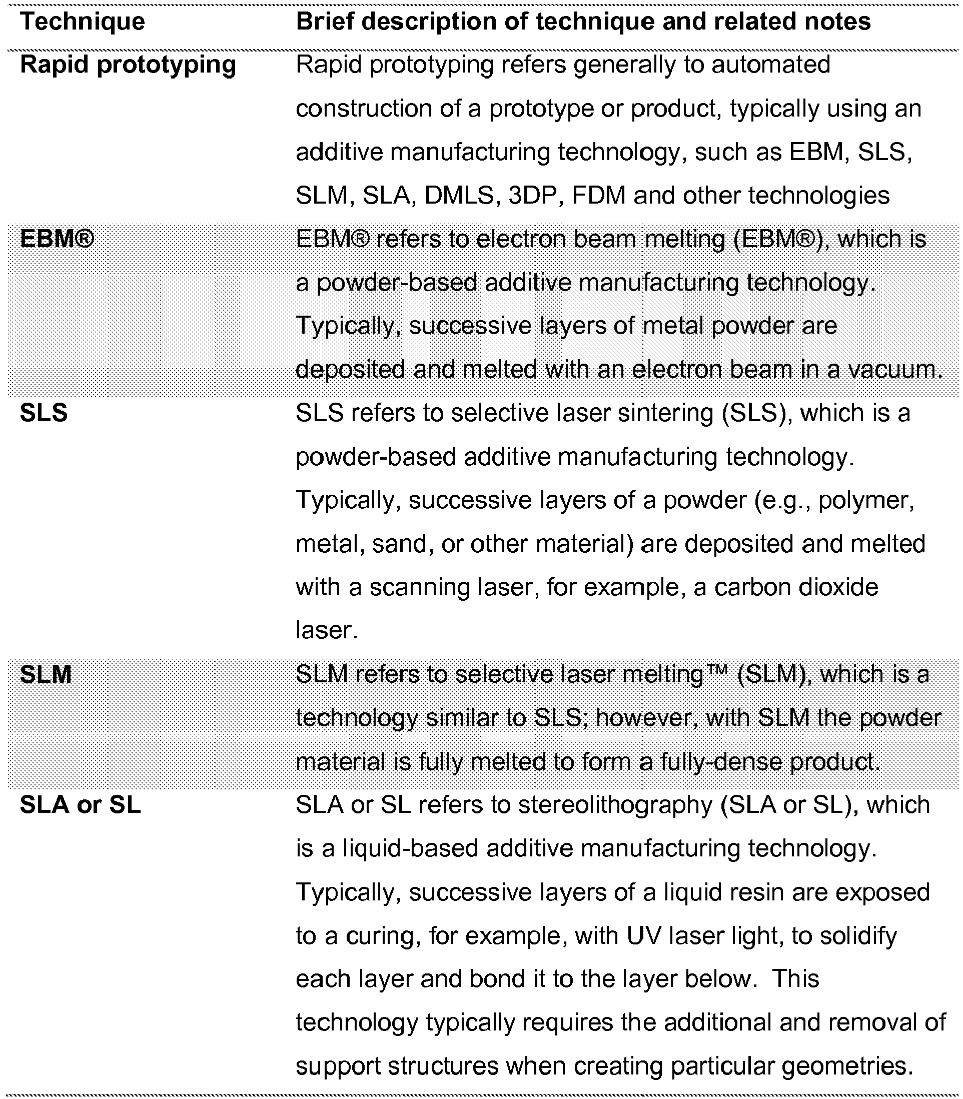

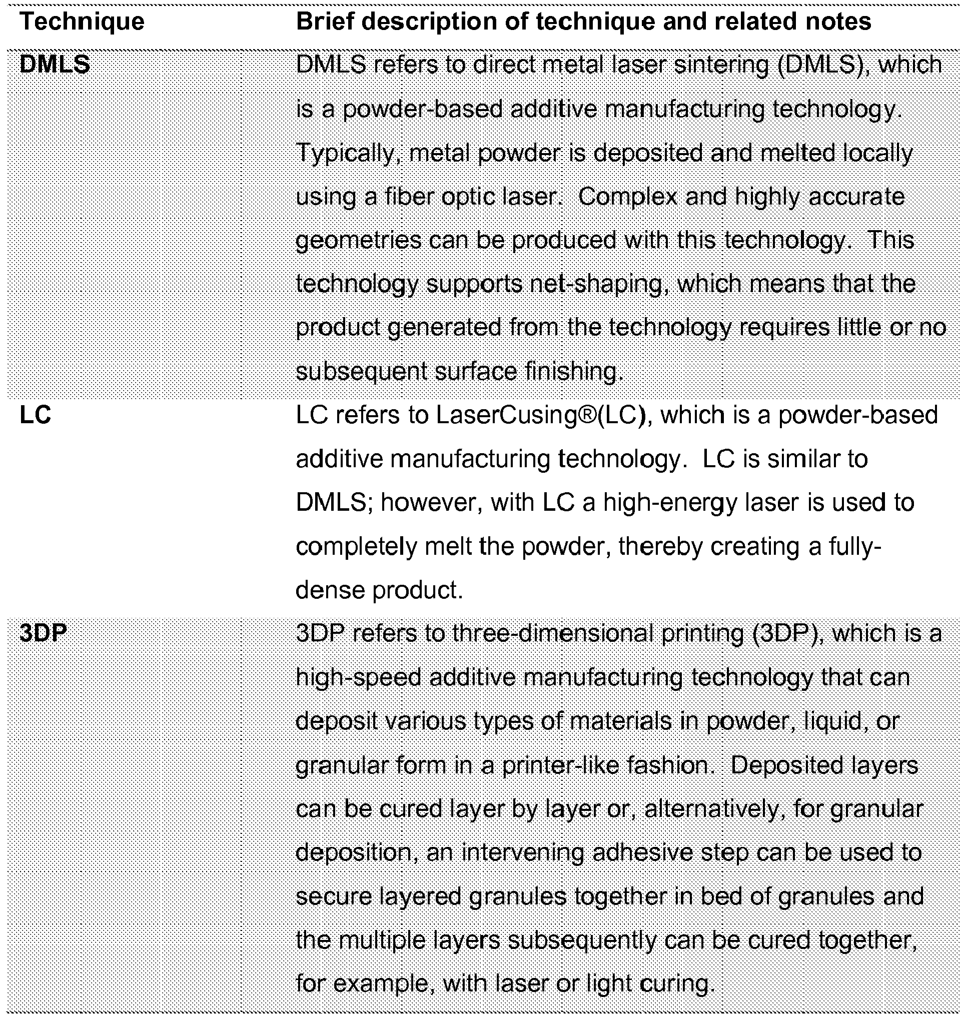

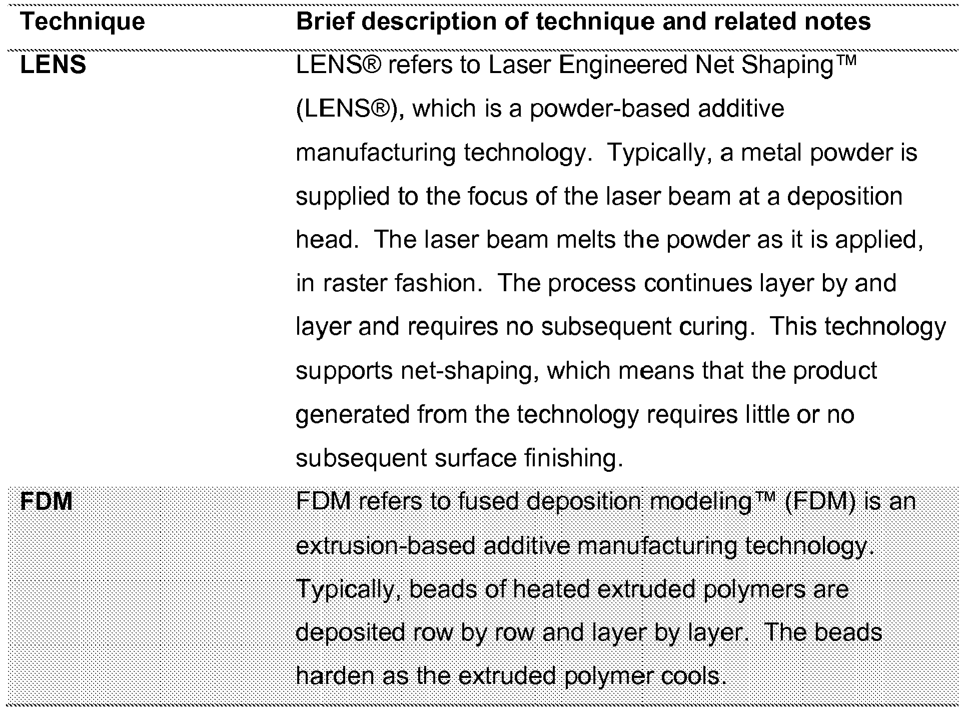

In the pursuit of patient-specific and/or patient-engineered implants, it would be extremely advantageous if one could employ 3-dimensional printing technology (also known as Solid Freeform Fabrication or "SFF") to create solid, physical implant components from an electronic or computerized data file (e.g., a CAD file). At first limited to the use of photo-curable polymers to create relatively fragile objects, 3D printing techniques have evolved over the past decades to allow the creation of prototypes, mould masters and/or "models" that could be used in metal casting techniques (e.g., lost-wax casting or investment casting). More recently, 3D printing techniques such as Selective Laser Sintering (SLS) and Selective Laser Melting (SLM - also known as Direct Metal Laser Sintering - DMLS - or LaserCusing) have been developed and refined to allow the creation of durable metallic objects, and it has been proposed that such techniques may allow the creation of biocompatible metal objects that could themselves directly serve as implant components.

As with any manufacturing process, including traditional processes such as the casting and/or forging of metals, the various advantages of different metal 3D printing techniques typically are accompanied by some disadvantages inherent with each of the manufacturing techniques. For example, while SLS allows a range of metallic systems and polymeric material choices in the creation of the physical object, SLS parts typically (1) have a rough grainy and porous surface finish, (2) experience a relatively large shrink rate causing the part to warp, bow or curl, and (3) have a surface feature detail that is relatively coarse. While a wider variety of materials is available for use in the SLM process, the technique as currently refined also comes with some associated disadvantages, including (1) high temperature gradients resulting in a build-up of thermal stresses, (2) a rapid solidification, leading to the occurrence of segregation phenomena and the presence of non-equilibrium phases, (3) the stability, dimensions and behavior of the particle "melt pool" determines to a great extent the porosity and surface roughness, and (4) the object has a surface roughness created by the layer-wise building techniques (e.g., the "staircase effect").

The steps of designing an implant component and/or guide tool, and associated methods of manufacturing such objects using additive material technologies such as SLM and SLS, as described herein, can include both configuring one or more features, measurements, and/or dimensions of the implant and/or guide tool (e.g., derived from patient-specific data from a particular patient and adapted for the particular patient), manufacturing and finishing the implant/tool. In certain embodiments, manufacturing can include making the implant component and/or guide tool from starting materials, for example, metals and/or polymers or other materials in solid (e.g., powders or blocks) or liquid form. In addition or alternatively, in certain embodiments, manufacturing can include altering (e.g., machining) an existing implant component and/or guide tool, for example, a standard blank implant component and/or guide tool or an existing implant component and/or guide tool (e.g., selected from a library), as well as post-manufacture machining and/or processing of an implant after manufacture by SLM techniques. The manufacturing techniques to making or altering an implant component and/or guide tool can include any techniques known in the art today and in the future. Such techniques include, but are not limited to additive as well as subtractive methods, i.e., methods that add material, for example to a standard blank, and methods that remove material, for example from a standard blank, as well as combinations thereof (i.e., using both additive and subtractive techniques on a single object). The design of an implant component and/or guide tool can include manufacturing, for example, using CAM software and additive, subtractive and/or casting manufacturing techniques as described herein.

In various embodiments, the design of an implant component and/or other manufactured object (e.g., a guide tool) may be altered or modified to accommodate advantages and/or limitations of a specific manufacturing process, such as SLM, which may result in differing designs for a single anatomical situation (e.g., for a single patient anatomy) based on differing manufacturing methods. The various design changes, which can (but not necessarily must) have varying degrees of impact on the ultimate performance and/or reliability of the implant, can be incorporated to accommodate a wide variety of considerations, including tolerancing and dimensioning limitations of specific manufacturing methodologies and/or equipment, design limitations and/or object feature (e.g., surface and/or subsurface feature) orientation and/or shape requirements, ease of object removal from manufacturing equipment and/or fixtures, ease of removing support surfaces or other ancillary artifacts from the manufacturing processes, improvements in manufacturing performance and/or manufacturability of multiple implants and/or implant components in a single machine "run" or batch, minimizing object and/or feature deformation and/or "warpage" during and subsequent to the manufacturing process, improving the repeatability and reliability of implant manufacturing processes and methods, and/or simplifying and/or improving the implant design to facilitate finishing and polishing of the object.

### SLM MANUFACTURING

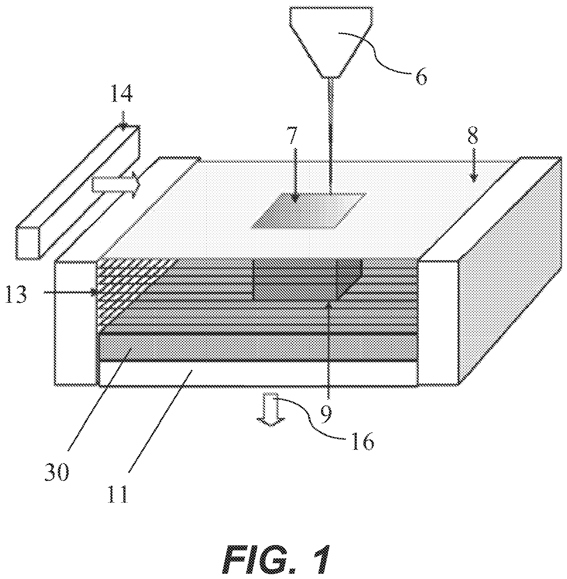

FIG. 1 depicts a schematic view of equipment and the process used in a typical SLM manufacturing process. SLM is a powder bed 8 process that begins with the deposition of a thin layer of powder onto a substrate 30, which can be disposed on a processing table 11. A high power laser 6 scans the surface of the powder, generating heat that causes the powder particles to melt (see melted powder 7) and form a melt pool which solidifies as a consolidated layer of material. Once the layer has been scanned and relevant portions melted/solidified, another layer of powder is deposited, which is then subsequently scanned and melted/solidified to form the next layer of the part. This process continues with multiple layers 13 until enough layers of material have been deposited/melted/solidified to create a desired object 9. Powder particles that are not melted remain loose and are removed (and can typically be reused) once the component is complete. Supports or other features/artifacts are typically required to anchor down certain unsupported features due to shrinkage and/or "curling" of solidifying material. To some extent, this anchoring requirement restricts the process of geometric freedom.

The production of parts using SLM has many difficulties. Many processing issues arise due to the use of a high power laser to fully liquefy material from a powder bed. High heat input often causes an increase in material vaporization and spatter generation during processing. Surface roughness is another SLM issue that is influenced by particle melting, melt pool stability and re-solidifying mechanisms. In addition, because the process involves the creation of localized melting and bonding of particles in a row, line by line, the possibility exists for "track instability" and/or "breaking up" of tracks associated with the formation of agglomerates and/or pores in the surface.

Various embodiments described herein include designs and methods to mitigate, reduce and/or eliminate structural and/or processing challenges and/or concerns posed by SLM manufacturing of implant components. In addition, various embodiments further include designs and methods that improve, maximize and/or take advantage of structural and/or processing benefits conferred by SLM manufacturing of implant components. In addition, various additional techniques, such as laser polishing or laser rescanning in parallel and/or perpendicular scanning directions (including remelting of previously formed surfaces and/or structures), hot isostatic processing (HIP) processing of manufactured implants, annealing and/or coating (e.g., titanium nitride coating and/or titanium aluminum nitride coating) are contemplated for use with the various embodiments disclosed herein.

In various embodiments, the SLM raw material comprises a CrCo powder having an average particle size of between 34 and 54 microns, although larger and/or smaller particles may used with varying degrees of utility (as well as the use of differing size particles in creating a single implant component). In various embodiments, the deposed particle layer may be approximately 60 microns thick, which, when melted, consolidated and cooled, can create a solid structural layer of approximately 20 microns thickness.

### ALIGNMENT AND ORIENTATION

Some significant features of various embodiments described herein include various methods, techniques and/or processes to align, orient and/or otherwise position implants or other objects to be manufactured relative to a known "home" or "zero" location and orientation of the SLM manufacturing equipment or portions thereof (e.g., the laser source and/or scanning mechanism). Consistent orientation and location relative to a known position can facilitate the reliable and repeatable manufacturing of implant components in a single machine and/or across multiple machines, and can further assist with identification and/or alleviation of process and/or design defects discovered during or after the manufacturing process. Moreover, proper alignment and/or location of a manufactured object relative to the manufacturing machinery allows a designer and/or operator to predict and/or accommodate for various manufacturing advantages and/or disadvantages inherent in the chosen manufacturing processes.

In one embodiment, an electronic design file (such as a CAD file, for example) for a knee implant component (in this example, a "total knee" implant component for replacing femoral surfaces) can be loaded into SLM processing equipment or otherwise accessed to facilitate manufacture of the component by the SLM equipment. The CAD design file can include a wide variety of information about the component, including desired outer surfaces for the implant. In some embodiments, the implant design can include information regarding various surfaces and/or other features of the implant, one or more of which can be designated as "reference parameters" for use in aligning and/or positioning the design and/or object relative to the SLM equipment.

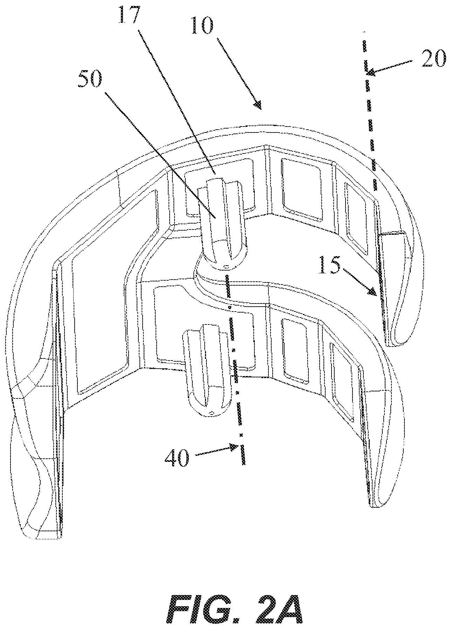

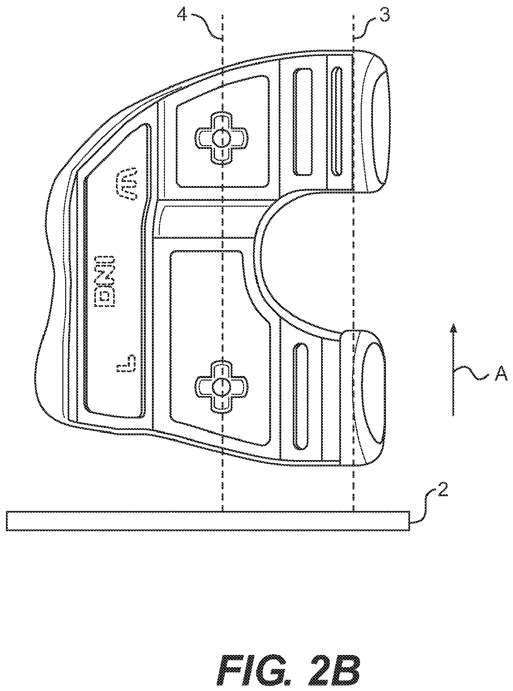

For example, in manufacturing a femoral component of a "total knee" implant, one embodiment (shown in FIG. 2A) includes the designation of a bone-facing implant surface 15 on a medial condylar portion 10 (e.g., facing a posterior bone cut on the medial condyle) of the implant as a first reference datum 20. This first reference datum 20 will desirably be aligned perpendicular to the object support structure or substrate 30 (see FIG. 1) that supports the object during the manufacturing process. In addition, the embodiment further includes the designation of a second reference datum 40, which can be a longitudinal axis of a support peg 50 or stem on the medial condylar portion 10 of the implant, which can be aligned parallel to the object support structure or substrate 30. Alternatively, the second datum could be determined using a distal bone-facing surface 17 of the implant, which can be aligned perpendicular to the object support structure or substrate 30. Designation of at least two datum 20 and 40 desirably define an orientation relative to the SLM equipment. In alternative embodiments, the datum may be aligned relative to the laser 6, the powder depositor and leveler 14, the powder bed 8, the processing table 11, the line of action of gravitational forces 16, or other relative measures. In various embodiments, one or more additional datum such as one or more known positions (e.g., one or more implant component positions, such as points where the post meets various implant surfaces) could be employed to further define object location and/or orientation. Another exemplary alignment configuration relative to platen 2 and build direction A, using a bone facing surface of the medial condylar portion as a first reference datum 3 and an axis passing through the support pegs as a second reference datum 4, is shown in FIG. 2B.

In various embodiments, the identification and employment of such datum in conjunction with SLM manufacturing equipment can ensure consistency throughout multiple "runs" of manufactured implants (in the same or different machines) and can significantly reduce time and/or effort (and possibly obviate a need for human intervention) required for "set up" of an individual SLM machine for creating a given implant design. Moreover, because various properties of the implant and its component material(s) can be dependent upon the direction and/or orientation of the implant feature(s) being created by the SLM equipment, due to a wide variety of factors, information regarding the proposed alignment of the object may be highly relevant to the implant design.

By choosing relative measures for alignment (e.g., bone-facing planar surfaces of the implant), various embodiments can define a repeatable alignment technique for the manufacture of patient specific implants of differing sizes and/or shapes via SLM techniques, which allows the designer to anticipate and/or accommodate various manufacturing considerations, limitations and/or advantages in designing and/or orienting the implant for manufacture.

### SUPPORT STRUCTURES

Many additive manufacturing processes and 3D printing methods require and/or prefer the use of support structures during part build. In many cases, the geometry cannot stand on its own, or the material requires support during melt and/or curing. In addition, the use of support structures can anchor the manufactured object within the manufacturing equipment, preventing the object from uncontrolled movement and/or rotation/displacement during the manufacturing process, which could potentially ruin and/or degrade the quality of the part. While various developers have experimented with supportless SLM manufacturing techniques, including the use of eutectic system alloys (materials which solidify at sharp temperature points), such processes have not yet been successful in high melt-temperature materials, including most medical-grade metals.

While support structures may be necessary during the manufacture of implant components, the supports are generally removed after manufacture and prior to finishing of the implant. Various embodiments described herein include improvements and/or modifications to standard SLM anchoring and/or support structures to facilitate the separation of the implant from the SLM equipment and/or substrate, the removal of such structures from the implant itself, and the design and/or placement of such support structures to minimize their impact on part quality and/or performance as well as any effects on finishing of the implant or other part.

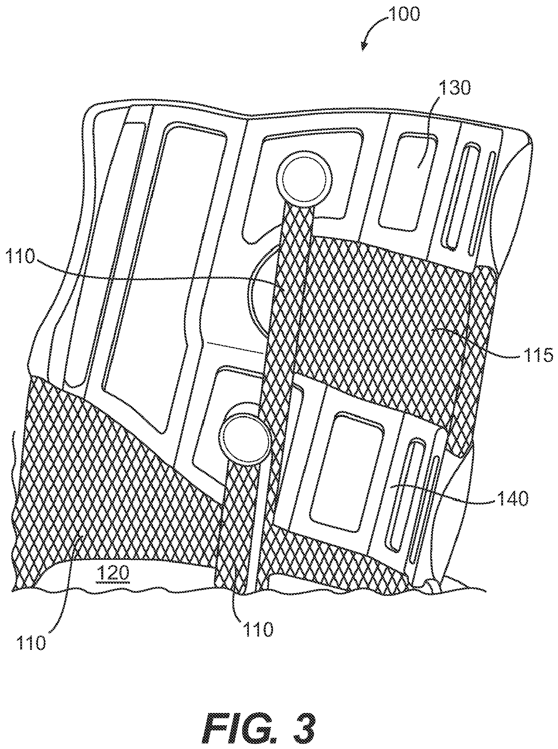

FIG. 3 depicts a perspective view of one embodiment of a femoral implant component 100 manufactured using a SLM manufacturing process. A series of support structures 110 extend between a support plate or substrate 120 and the implant 100. Additional support structures 115 extend between the medial condylar portion 130 and the lateral condylar portion 140 of the implant 100.

In various embodiments, the location of support structures and their respective attachment points can be positioned to avoid and/or minimize contact with specific portions of the implant, if possible. For example, the inner, bone-facing surfaces of a femoral implant component (including the inner surfaces of the "cement pockets" and/or bone ingrowth surfaces) are often structures that do not require significant "finishing" after manufacture (and/or the need for such finishing is not desired by the manufacturer). Where SLM support structures contact and/or extend outward of such component surfaces, their detachment and removal may necessitate additional processing and/or finishing of such surfaces, which may be difficult to perform (e.g., the surfaces may recessed and/or obstructed by other surfaces and/or structures) or simply involve additional, unnecessary expense. By avoiding support contact with such non-finished or minimally-finished surfaces, the time and expense associated with implant manufacture can be minimized.

In various embodiments, the location of support structures and their respective attachment points can be positioned to avoid and/or minimize contact with surfaces intended for implant articulating or other surfaces where surface dimensionality and/or shape are critical or important features of the implant. For example, the outer, joint facing surfaces of a femoral implant component (especially those directly opposite to the inner, bone-facing surfaces) typically form articulating surfaces that interact with polymer and/or metal surfaces of opposing implant components. Where SLM support structures contact and/or extend outward of such articulating surfaces, their detachment and removal may necessitate additional processing and/or finishing of such surfaces, especially where the presence of the support structures have increased the local porosity of the material. Moreover, removal of the support structures and finishing of the articulating surfaces may necessitate the removal and polishing off of significantly more implant material, potentially altering the carefully designed shape of the articulating surface as well as involving additional, unnecessary expense. By avoiding support contact with such non-finished surfaces, the time and expense associated with implant manufacture can be minimized.

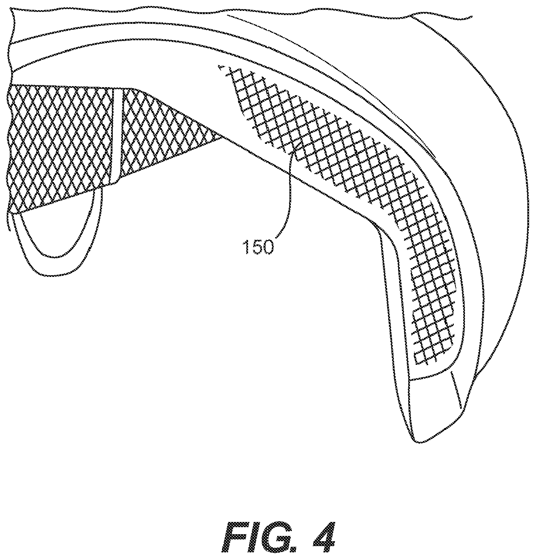

In various embodiments, the support structures and their respective attachment points can be positioned adjacent to peripheral edges of the implant, as well as between adjacent peripheral edges of the medial and lateral condylar portions of the implant. In this manner, the effects of the support structures on critical aspects of the implant (e.g., intended articulating surfaces and/or bone-facing surfaces), and the amount of effort required to remove and finish surfaces associated with support structures, can be minimized. Moreover, in various embodiments, support structures may not be used (or may be used sparingly) (1) in confined areas (e.g., deep within the intercondylar notch) or (2) in areas having surface features that render removal of the support structures and subsequent finishing difficult or time consuming (e.g., along highly curved surfaces or within recessed areas). FIG. 4 depicts one exemplary femoral implant incorporating support structures (removed in the image) on a peripheral edge 150, which in various embodiments can simplify removal and finishing of the various implant surfaces (including the inner surfaces, outer articulating and peripheral edge surfaces).

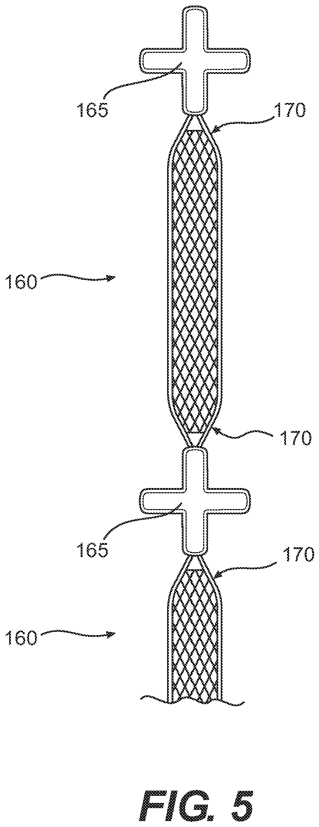

In various embodiments, the support structures can incorporate various design features to simplify their removal from the manufactured implant. For example, FIG. 5 depicts a side view of support structures 160 extending between cross-shaped implant posts 165, with the support structures 160 including areas of significantly reduced cross-section 170. The area of reduced cross-section can be configured to provide sufficient support to the attached surfaces of the implant to accomplish the twin goals of supporting and/or anchoring the surface, while facilitating cutting or other separation of the support structure after SLM manufacture. In certain embodiments, such as where appropriate thickness, design and structural considerations have been achieved, the reduced cross-section area may function as a frangible link or break-away tab.

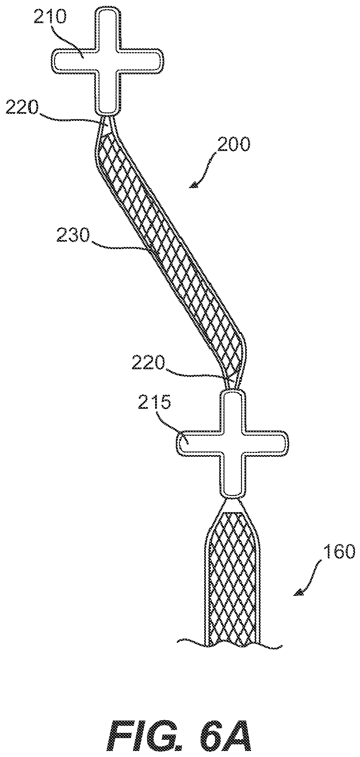

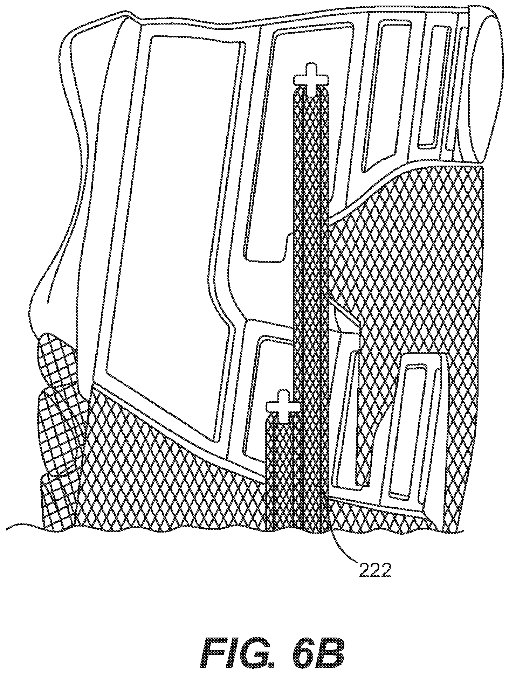

FIG. 6A depicts an alternative embodiment of a support structure 200, where the structure 200 supports an implant post 210 that is laterally spaced from another implant post 215 during manufacture. Similar to the embodiment previously described, the structure 200 includes areas of reduced cross-section 220 to facilitate removal of the support structure after manufacture. This embodiment further includes an inclined or titled section 230 that angles or shifts laterally to properly support the superior post 210. During SLM manufacture, support structures are often utilized to anchor or otherwise secure object features to minimize deformation of various features during the melting/cooling/consolidation process. In various situations, it may be desirous to use such angled or laterally-spaced anchors, or similar support structures, to secure the relevant object features to other object features, rather than simply extend an individual support structure the entire way to the substrate or support platform (as shown by support 222 in FIG. 6B).

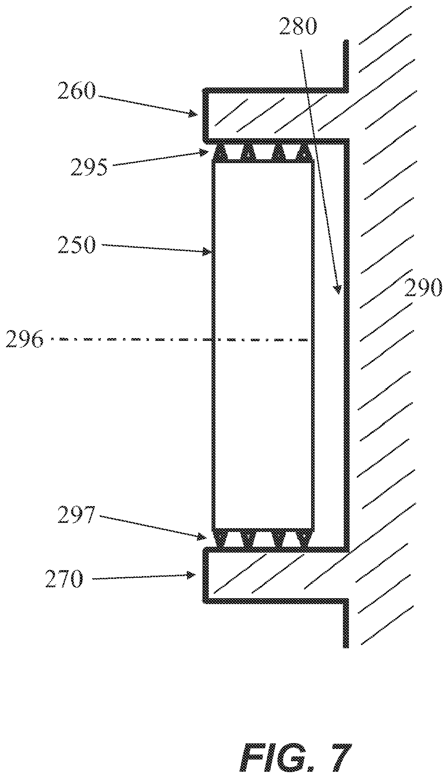

FIG. 7 depicts another embodiment of a support structure where the structure 250 extends between two implant posts 260 and 270. In this embodiment, a significant portion of the support structure is spaced away from an adjacent surface 280 of the manufactured object 290, with the ends of the structure connected via reduced cross-section sections 295 and 297 to the respective implant posts 260 and 270. Desirably, this embodiment will provide sufficient support to prevent warpage of the posts, while reducing and/or obviating the need to separate the support structure from the surface 280 and/or require additional finishing of the surface 280 when manufacture is complete. In various embodiments, the support structure is spaced apart from the surface 280 by at least 0.25 mm, although various other spacing arrangements may be utilized with varying utility.

The support structure arrangement of FIG. 7 can further facilitate the removal of the support structure in a safe and efficient manner. For example, if desired, a rotary cutting tool or "tin-snip" device could be utilized to cut the central region along line 296, and then a grasping device such as a pair of pliers could be used to grasp the individual support structure halves and rotate the structures such that the reduced cross-section areas 295 are flexed and/or "work-hardened" (in a known manner), thereby causing the reduced cross-sectional areas to fracture and allow removal of the support structure quickly and without requiring the use of cutting tools close to other surfaces of the object (which may include surfaces that could be damaged and/or ruined through inadvertent contact with the cutting tool).

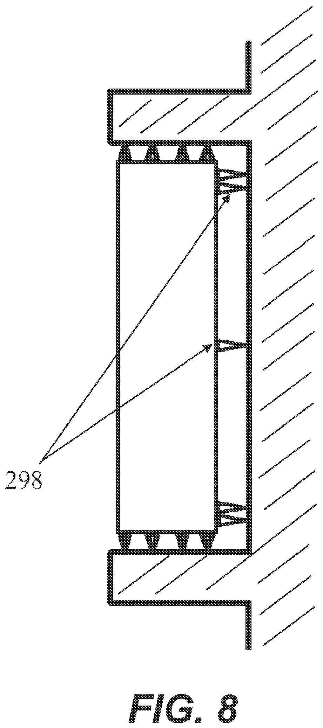

FIG. 8 depicts an alternate embodiment of the support structure of FIG. 7, with the addition of lateral reduced cross-section attachment points 298, which may be required in various embodiments to provide additional structural support. In various embodiments, the use of few lateral supports (or the absence of such lateral or other supports) may allow various features of the implant and/or the support structures to "pull away," deform and/or separate from each other, which may be desirable in certain situations, especially where such movement and/or fracture facilitates removal of the support without appreciably affecting the ultimate shape of the implant feature (or where the implant feature already requires further "finishing"). In such a case, the support structure may be intentionally designed to cause such movement and/or fracture during the cooling process.

In various embodiments, the manufactured object may be supported a desired distance above the substrate or platform, to allow sufficient clearance for a wide variety of cutting and/or removal tools. For example, where a pair of "tin-ships" or other similar cutting devices (e.g., metal shears, dikes, pliers, etc.) are used to sever the support structure between a femoral implant component and the substrate, it may be desirous to ensure that a spacing of 1 cm or more of clearance exists between the substrate and the lowest point of the implant.

### PEG/POST DESIGNS

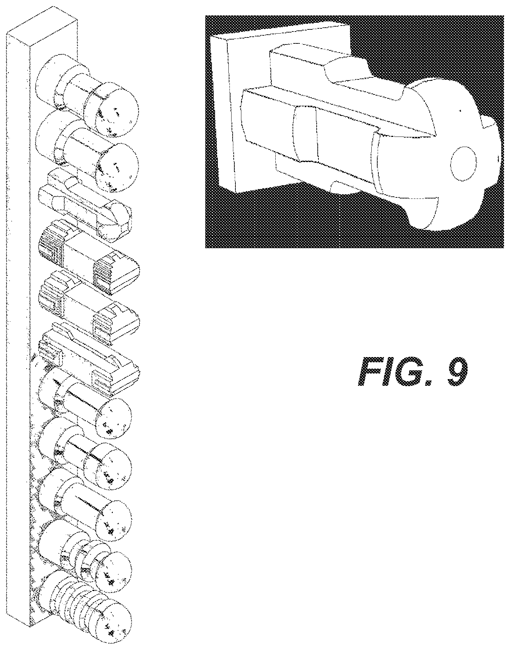

In certain embodiments, various design features of the implant and/or its supporting structures may be altered, modified or particularized for a desired manufacturing method. For example, in the case of a femoral implant component manufactured using SLM manufacturing techniques, it may be desirous to modify the peg or post designs to include regular-shaped pegs with few voids or inclusions therein (e.g., cross-shaped, cylindrical, triangular, rectangular and/or other regular geometric peg shapes). Alternatively, it may be desirous to incorporate complex peg designs that include varying quantities of voids and/or inclusions for a wide variety of reasons, including to act as bone ingrowth and/or bone cement retention structures and/or surfaces. FIG. 9 depicts various complex geometries that may be utilized in the design and creation of such pegs or posts.

In a similar manner, the various manufacturing techniques described herein, such as SLS or SLM manufacturing, may be utilized to create complex geometries and/or surfaces that can be employed for a variety of functions, which could include the creation of textured and/or porous-walled cement pockets and/or bony ingrowth surfaces, for securing the implant to the patient's underlying bone. Various shapes could include defined micro-cavities and/or micro-protrusions on the implant surface.

In various embodiments, the pegs may be designed to obviate the need for manufacturing support structures, such as where the pegs are conically shaped or formed in similar shapes. If accuracy, shape and/or dimensions of the pegs are not a critical factor, or where post manufacturing machining of the pegs is performed (or where the pegs are installed in the post-manufacturing phase, such as by drilling and tapping the implant and installing threaded pegs), the use of support structures for the pegs may not be mandated and/or necessary.

### ACCOMMODATING STRESSES AND FEA ANALYSIS

In various embodiments, the design of a given implant component and/or various features therein can be further assessed and/or modified by including FEA modeling and/analysis, either alone or in combination with information relating to the specific manufacturing method chosen for creating the implant. For example, the creation of an implant using SLM manufacturing methods may produce an implant having differing density, porosity, durability, fatigue strength and/or other material properties than those of an implant created through traditional casting techniques. A finite element analysis (FEA) of an SLM implant and/or intended implant design may identify areas of the implant/design prone to increased and/or excessive loads, which may induce the designer to modify the design to better accommodate the anticipated loading (e.g., increase the local or global implant thickness and/or alter implant geometry or location of planar surfaces).

In various embodiments, the design and/or orientation of an implant may be modified and/or altered due to various features of the manufacturing method(s). For example, in SLM manufacturing, the mechanical properties in SLM parts can be anticipated to be anisotropic mainly due to the fact that part build-up is conducted with many melted tracks (or vectors) and layers melted onto each other. Solidification microstructure of SLM parts generally determines the strength properties, and the solidification microstructure essentially depends on the local solidification. Moreover, notable thermal stresses may exist in SLM parts because of the large temperature gradients caused by the rapid cooling during the SLM processing. In addition, SLM parts may have a greater level of elasticity within an individual layer than between layers, which may result in crack propagation and/or cleavage at layer contacts.

Various embodiments disclosed herein include the modification of implant designs, manufacturing orientations and/or manufacturing methods to accommodate mechanical properties of implant components manufactured using SLM methodologies. For example, in designing an implant component, and then orienting that component for manufacture using SLM, it may be desirous to minimize the potential for crack propagation and/or cleavage inducing a complete or catastrophic failure of the implant.

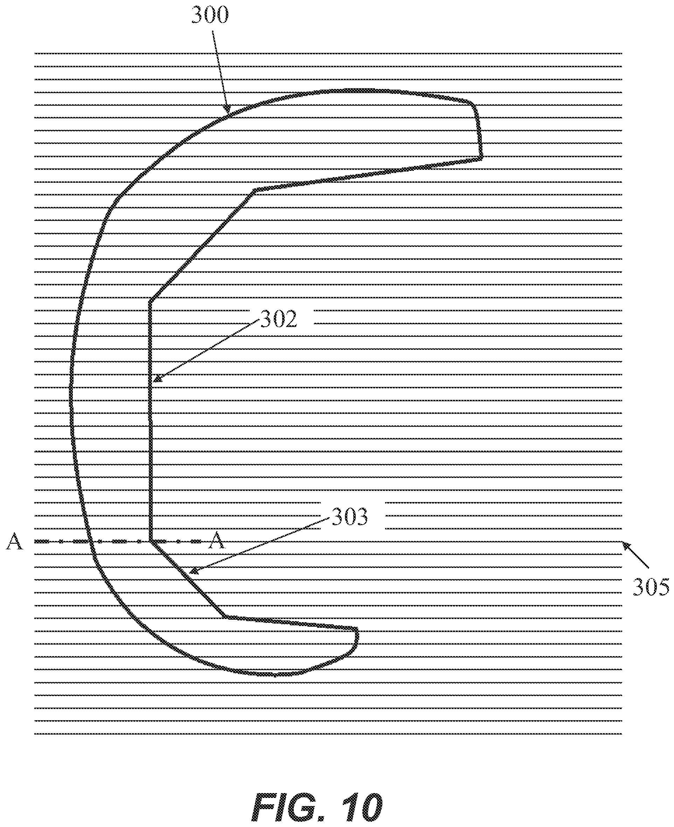

FIG. 10 depicts a cross-sectional view of an exemplary embodiment of an implant design and manufacturing orientation that could potentially result in an increased likelihood of implant failure. The implant 300 has been manufactured using a SLM process, with a plurality of horizontal layers (shown as the parallel horizontal lines in the figure, which is a greatly simplified representation of the numerous layers used to create the implant) representing the SLM manufacturing process. An FEA or other analysis of the implant, which optionally may include material property information particular to the type of manufacturing processes as well as the design and orientation of the implant, may identify one or more locations of high stress and/or areas of localized implant weakness. One such region that could be prone to fracture and/or failure could be a portion of the implant in the vicinity of region A-A, which includes a region where the minimum implant thickness (at a planar boundary between planar surfaces 302 and 303) approximately meets a horizontal layer 305 created during the SLM manufacturing process. In such a case, it may be desirous to increase the local implant thickness proximate this region and/or alter the orientation of the implant during manufacture to reduce the fracture potential along this layer.

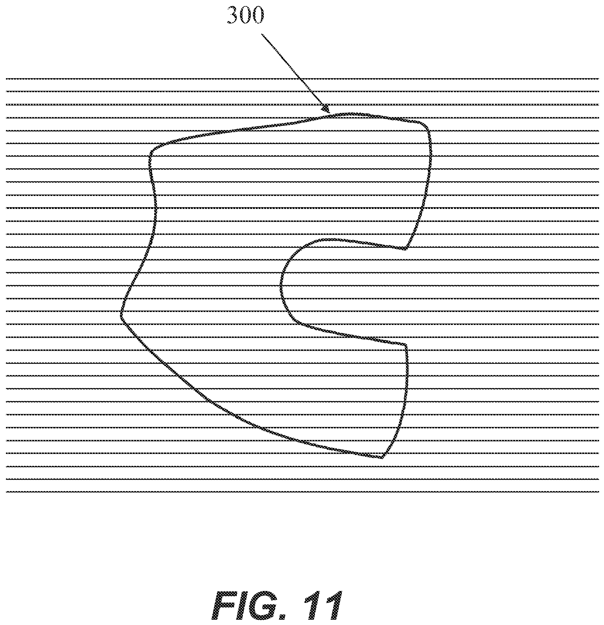

FIG. 11 depicts a side view of the implant of FIG. 10, with a modified manufacturing orientation that desirably results in a decreased likelihood of implant failure along a given manufacturing layer (as compared to the embodiment of FIG. 10). The implant 300, which has been manufactured using a SLM process, incorporates a plurality of horizontal layers (shown as the parallel horizontal lines in the figure, which is a greatly simplified representation of the numerous layers used to create the implant) representing the SLM manufacturing process. An FEA or other analysis of the implant, which optionally may include material property information particular to the type of manufacturing processes as well as the design and orientation of the implant, may be less likely to identify one or more locations of high stress and/or areas of localized implant weakness that are concurrent with manufacturing layers or other artifacts inherent in the manufacturing processes. In such a case, it may be possible to decrease the local implant thickness in various regions and/or further optimize the orientation of the implant during manufacture to reduce any fracture potential. By incorporating potentially weaker areas of the implant along the longitudinal axis of the condylar portions, the present embodiment may be less likely to fail, or may be designed to fail in less critical regions.

In various embodiments, the use of multiple alignments and/or orientations to create a single implant component is contemplated. For example, where FEA analysis of a part design and/or orientation identifies multiple regions of potential weakness, and redesign/reorientation of the design prior to SLM manufacture does not sufficiently alleviate strength and/or durability concerns, it may be desirous to reposition and/or reorient the object at one or more times part-way through the manufacturing process (e.g., pausing the layer deposition and laser melting process, moving/rotating the partly finished implant in some manner, and then continuing the layer deposition and laser melting process to complete the implant manufacture) to address localized fracture potential.

### ADDITIONAL PROCESSING STEPS

In various embodiments, the design and manufacture of an implant component using SLM manufacturing methods may include additional processing and/or finishing steps which are not mandated, required and/or are necessary to prepare the part for use and implantation when the part is manufactured using conventional methods (e.g., casting, wrought and/or machining, etc.). For example, if the surface porosity of an implant created via SLM manufacturing is unacceptable fro a given application, it may be necessary to remove and/or fill the pores using a variety of additional manufacturing and/or finishing steps, which can include coating, filling, remelting, HIP-ping, annealing and/or machining, as well as potentially adding additional material to the implant surface to thereby allow for additional polishing and/or grinding to remove the undesired surface features. Various embodiments described herein include the use of such additional processes to "finish" a SLM part, including the use of such processes on a localize portion of the implant (e.g., performed only on the articulating surfaces or other implant surfaces having undesirable features or characteristics).

In various embodiments, a wide variety of standard finishing techniques used with cast or wrought implants, such as polishing, drag finishing, machining and/or bead/grit blasting, may be used to finish SLM parts as well, with varying results.

### ACCOMMODATING DIFFERENT MANUFACTURING METHODS

Implant components generated by different techniques can be assessed and compared for their accuracy of shape relative to the intended shape design, for their mechanical strength, and for other factors. In this way, different manufacturing techniques can supply another consideration for achieving an implant component design with one or more target features. For example, if accuracy of shape relative to the intended shape design is critical to a particular patient's implant component design, then the manufacturing technique supplying the most accurate shape can be selected. If a minimum implant thickness is critical to a particular patient's implant component design, then the manufacturing technique supplying the highest mechanical strength and therefore allowing the most minimal implant component thickness, can be selected. Branner *et al.* describe a method a method for the design and optimization of additive layer manufacturing through a numerical coupled-field simulation, based on the finite element analysis (FEA). Branner's method can be used for assessing and comparing product mechanical strength generated by different additive layer manufacturing techniques, for example, SLS, SLM, DMLS, and LC.

In certain embodiments, an implant can include components and/or implant component parts produced via various methods. For example, in certain embodiments for a knee implant, the knee implant can include a metal femoral implant component produced by casting or by an additive manufacturing technique and having a patient-specific femoral intercondylar distance; a tibial component cut from a blank and machined to be patient-specific for the perimeter of the patient's cut tibia; and a tibial insert having a standard lock and a top surface that is patient-specific for at least the patient's intercondylar distance between the tibial insert dishes to accommodate the patient-specific femoral intercondylar distance of the femoral implant.

As another example, in certain embodiments a knee implant can include a metal femoral implant component produced by casting or by an additive manufacturing technique that is patient-specific with respect to a particular patient's M-L dimension and standard with respect to the patient's femoral intercondylar distance; a tibial component cut from a blank and machined to be patient-specific for the perimeter of the patient's cut tibia; and a tibial insert having a standard lock and a top surface that includes a standard intercondylar distance between the tibial insert dishes to accommodate the standard femoral intercondylar distance of the femoral implant.

### TIBIAL TRAYS

In a manner similar to the various embodiments described herein in connection with femoral implant components, a tibial tray component can be machined, molded, casted, manufactured through additive techniques such as laser sintering, selective laser melting or electron beam melting or otherwise constructed out of a metal or metal alloy such as cobalt chromium. Similarly, the insert component may be machined, molded, manufactured through rapid prototyping or additive techniques or otherwise constructed out of a plastic polymer such as ultra high molecular weight polyethylene. Other known materials, such as ceramics including ceramic coating, may be used as well, for one or both components, or in combination with the metal, metal alloy and polymer described above. It should be appreciated by those of skill in the art that an implant may be constructed as one piece out of any of the above, or other, materials, or in multiple pieces out of a combination of materials. For example, a tray component constructed of a polymer with a two-piece insert component constructed one piece out of a metal alloy and the other piece constructed out of ceramic.

### OTHER JOINTS

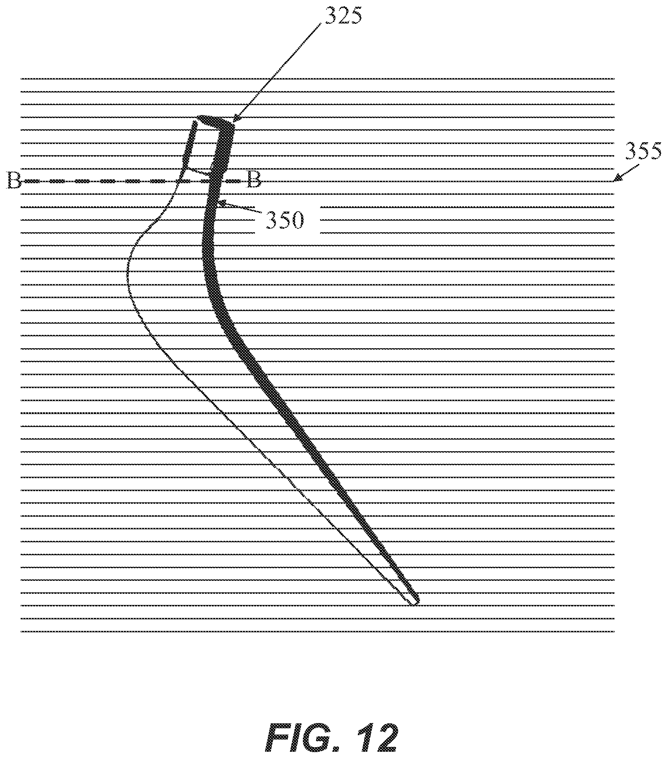

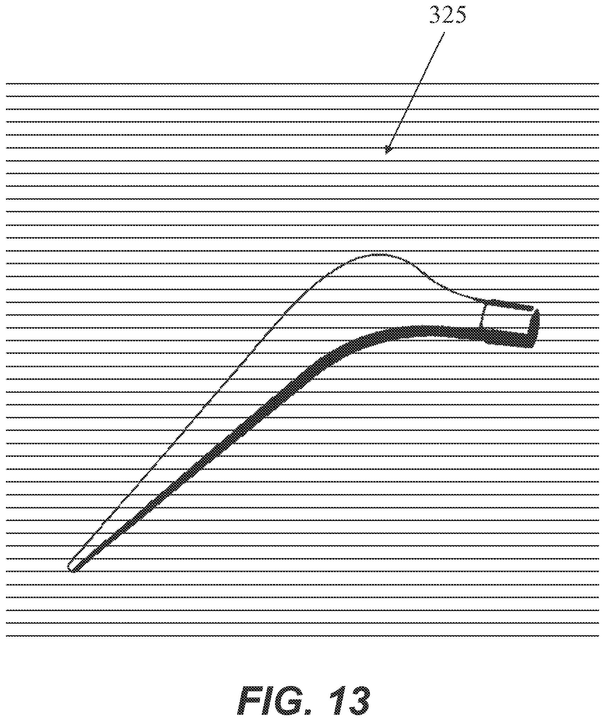

While the various embodiments and teachings therein are described with regards to a knee joint, the various embodiments described herein can be applied to various other joints or joint surfaces in the body, e.g., a knee, hip, ankle, foot, toe, shoulder, elbow, wrist, hand, and a spine or spinal joints. For example, the material properties of a hip stem manufactured using SLM techniques may similar be dependent upon the orientation and/or alignment of the design during manufacture. FIG. 12 depicts a hip stem 325 and a plurality of horizontal layers (shown as the parallel horizontal lines in the figure, which is a greatly simplified representation of the numerous layers used to create the implant) representing the SLM manufacturing process. An FEA or other analysis of the implant, which optionally may include material property information particular to the type of manufacturing processes as well as the design and orientation of the implant, may identify one or more locations of high stress and/or areas of localized implant weakness. One such region that could be prone to fracture and/or failure could be a portion of the implant in the vicinity of region B-B, which includes a region where maximum implant stresses (for example, along the neck 350 of the implant) approximately meets a horizontal layer 355 created during the SLM manufacturing process. In such a case, it may be desirous to increase the local implant thickness proximate this region and/or alter the orientation of the implant during manufacture to reduce the fracture potential along this layer. FIG. 13 depicts the same hip stem 325, where rotation of the implant design during manufacture could potentially reduce the potential for such neck fractures due to localized material conditions and/or fracture planes.

### MATERIALS

Any material known in the art can be used for any of the implant systems and component described in the foregoing embodiments, for example including, but not limited to metal, metal alloys, combinations of metals, plastic, polyethylene, cross-linked polyethylene's or polymers or plastics, pyrolytic carbon, nanotubes and carbons, as well as biologic materials.

Any fixation techniques and combinations thereof known in the art can be used for any of the implant systems and component described in the foregoing embodiments, for example including, but not limited to cementing techniques, porous coating of at least portions of an implant component, press fit techniques of at least a portion of an implant, ingrowth techniques, etc.

### INCORPORATION BY REFERENCE

The entire disclosure of each of the publications, patent documents, and other references referred to herein is incorporated herein by reference in its entirety for all purposes to the same extent as if each individual source were individually denoted as being incorporated by reference.

### EQUIVALENTS

The invention may be embodied in other specific forms without departing from the spirit or essential characteristics thereof. The foregoing embodiments are therefore to be considered in all respects illustrative rather than limiting on the invention described herein. The true scope of the invention is thus indicated by the descriptions contained herein, as well as all changes that come within the meaning and ranges of equivalency thereof.

### DISCLOSED ITEMS

1. A method of additive manufacturing of a patient-adapted femoral implant for a knee joint of a patient, the femoral implant having one or more articular surfaces, one or more bone-facing surfaces, one or more peripheral edges, and one or more pegs configured to extend into at least one condyle of the knee, the method comprising:
   aligning a design for the implant relative to a substrate in a manufacturing apparatus;
   providing one or more support structures for supporting one or more portions of the implant; and
   detaching the one or more support structure from the implant,
   wherein the one or more support structures does not contact the one or more articular surfaces,
   wherein the one or more support structures contacts the one or more pegs, and
   wherein the one or more support structures is positioned and/or oriented to avoid contacting adjacent surfaces of the implant, other than the one or more portions of the implant to be supported.
2. The method of item 1, wherein the one or more support structures does not contact the one or more bone-facing surfaces.
3. The method of item 1, wherein the one or more support structures contacts the one or more pegs.
4. The method of item 1, wherein the one or more support structures is positioned and/or oriented such that it is spaced at least about 0.25 mm from adjacent surfaces of the implant, other than the one or more portions of the implant to be supported.
5. The method of item 1, wherein the one or more support structures is configured to support powder to be melted and/or cured.
6. The method of item 1, wherein the one or more support structures is configured to anchor the implant to the substrate during manufacturing to substantially prevent uncontrolled movement of the implant.
7. The method of item 1, wherein the one or more support structures is configured to anchor the implant to the substrate during manufacturing to prevent, at least partially, deformation of the implant during a process selected from the group of processes consisting of melting, cooling, consolidating, and combinations thereof.
8. The method of item 1, wherein the aligning further comprises orienting the design for the implant relative to the substrate such that the strength of the implant will be increased, relative to other potential orientations, at one or more portions of the implant predetermined to be subject to high stress and/or localized weakness.
9. The method of item 1, further comprising performing a finite element analysis of the design for the implant, and wherein the aligning is based, at least in part, on the finite element analysis.
10. The method of item 1, wherein a portion of the one or more support structures that contacts the implant has a cross-sectional area that is smaller than a cross-sectional area of another portion of the one or more support structures.
11. The method of item 1, wherein the aligning includes aligning a bone-facing surface of the design for the implant substantially perpendicular to the substrate.
12. A method of additive manufacturing of an implant, the implant having one or more articular surfaces, the method comprising:
   aligning a design for the implant relative to a substrate in a manufacturing apparatus;
   providing one or more support structures contacting one or more portions of the implant to be supported; and
   removing the support structure from the implant,
   wherein the one or more support structures do not contact the one or more articular surfaces.
13. The method of item 1 or the method of item 12, wherein the additive manufacturing comprises a technique selected from the group of manufacturing techniques consisting of electron beam melting, selective laser sintering, selective laser melting, stereolithography, direct metal laser sintering, three-dimensional printing, fused deposition modeling, laser curing, and laser engineered net shaping.

## Claims

1. A support structure for use in an additive manufacturing process of making an implant, comprising:
one or more design features configured to facilitate removal of the support structure from the manufactured implant,
wherein said one or more design features include areas of significantly reduced cross-section of the support structure and are configured to support and/or anchor one or more surfaces of the implant during the 3D printing process.

2. The support structure of claim 1, wherein the manufactured implant includes an articular surface, wherein the support structure is configured not to contact the articular surface.

3. The support structure of claim 1, wherein the manufactured implant includes a bone-facing surface, wherein the support structure is configured not to contact the bone-facing surface.

4. The support structure of claim 1, wherein the support structure is configured to anchor the implant to the substrate during manufacturing to prevent, at least partially, deformation of the implant during a process selected from the group of processes consisting of melting, cooling, consolidating, and combinations thereof.

5. The support structure of claim 1, wherein a portion of the support structure that contacts the implant has a cross-sectional area that is smaller than a cross-sectional area of another portion of the support structure.

6. The support structure of claim 1, wherein the additive manufacturing comprises a technique selected from the group of manufacturing techniques consisting of electron beam melting, selective laser sintering, selective laser melting, stereolithography, direct metal laser sintering, three-dimensional printing, fused deposition modeling, laser curing, and laser engineered net shaping.
